# EUROPÄISCHE PATENTSCHRIFT

(11) **EP 0 707 593 B1**
(45) Veröffentlichungstag und Bekanntmachung des Hinweises auf die Patenterteilung: **29.12.1997**
(21) Anmeldenummer: 94917548.3
(22) Anmeldetag: 15.06.1994
(51) Int. Cl.: C07J 53/00, A61K 31/565

(54) **ORAL ESTROGEN WIRKSAME ESTER DES 14 ALPHA,15 ALPHA-METHYLEN-ESTRADIOLS**
ORALLY ADMINISTERABLE ESTERS OF 14 ALPHA,15 ALPHA-METHYLENE-OESTRADIOL WITH AN OESTROGENIC ACTION
ESTERS DU 14 ALPHA,15 ALPHA-METHYLENE- STRADIOL A ACTION STROGENE ADMINISTRABLES PAR VOIE ORALE

(30) Priorität: 03.07.1993 DE 4322186
(43) Veröffentlichungstag der Anmeldung: 24.04.1996
(73) Patentinhaber: Jenapharm GmbH & Co. KG, 07745 Jena (DE)
(72) Erfinder: SCHWARZ, Sigfrid, D-07743 Jena (DE); SIEMANN, Hans-Joachim, D-07747 Jena (DE); HENKEL, Harry, D-99510 Apolda (DE); PONSOLD, Kurt, D-07743 Jena (DE); DITTGEN, Michael, D-99510 Apolda (DE); FRICKE, Sabine, D-00749 Jena (DE); OETTEL, Michael, D-07743 Jena (DE); KAUFMANN, Günter, D-07743 Jena (DE); ELGER, Walter, D-14195 Berlin (DE); SCHNEIDER, Birgit, D-07745 Jena (DE); STÖLZNER, Wolfgang, D-07745 Jena (DE)
(74) Vertreter: Wablat, Wolfgang, Dr.Dr.
(86) Internationale Anmeldenummer: DE9400662
(87) Internationale Veröffentlichungsnummer: WO9501988

(56) Entgegenhaltungen:
- DE-A- 4 239 946
- FR-A- 2 429 797
- GB-A- 985 634
- JOURNAL OF STEROID BIOCHEMISTRY, Bd.29, Nr.1, 1988 Seiten 1 - 8 M. GYLING ET AL 'Estrogen and Antiestrogen Interaction with Estrogen Receptor of MCF-7 Cells-Relationship between Processing and Estrogenicity'
- JOURNAL OF STEROID BIOCHEMISTRY, Bd.25, Nr.5A, 1986 Seiten 677 - 682 S. STOESSEL ET AL 'Competitive Binding Assay for Estrogen Receptor in Monolayer Culture: Measure of Receptor Activation Potency'
- CHEMICAL ABSTRACTS, vol. 101, no. 10, 3. September 1984, Columbus, Ohio, US; abstract no. 78827, J. C. BROSSE ET AL 'Topical Formulations Containing Estrane Derivatives' Seite 379 ;Spalte 1 ; & FR,A,2 531 089 (LABORATOIRES PHARMASCIENCE) 3. Februar 1984
- R. A. HILL ET AL 'DICTIONARY OF STEROIDS' 1991 , CHAPMAN & HALL , LONDON, GB siehe Nr E-00310 siehe Seite 414 - Seite 416
- Webster's Third New International Dictionary Band 1, 1976. G&C Merriam Co, pp 778

## Beschreibung

Die Erfindung betrifft oral estrogen wirksame Ester des 14α 15α-Methylen-estradiols, die eine genau definierte Estrogenwirkung im Vergleich zum Ethinylestradiol oder zu natürlichen Estrogenen, vorzugsweise unter oraler Applikation, aufweisen.

Estrogene sind hormonale Substanzen, die in die Regulation der meisten Sexualfunktionen und vieler Stoffwechsel funktionen involviert sind. Estrogene können aus definierten Vorstufen in verschiedenen Organen und Geweben des Menschen synthetisiert werden.

Bei der erwachsenen Frau stammt der wesentliche Anteil der im Blut zirkulierenden Estrogene aus den Ovarien. Fällt diese Sekretion aus, z. B. durch die medikamentöse Hemmung der Bildung neuer Follikel oder durch den spontanen Ausfall der Follikelreifung, so drohen Mangelzustände, denen z. T. Krankheitswert zukommt.

Diese betreffen die Störungen der Befindlichkeit, Störungen verschiedener Kreislauffunktionen, ungünstige Veränderungen der Lipidspiegel des Blutes im Hinblick auf die Entwicklung einer Arteriosklerose und Abbauvorgänge im Knochen bis hin zur manifesten Osteoporose.

Der menstruelle Zyklus kommt mit dem Ende der ovariellen Hormonproduktion zum Erliegen. Wird die Ovulation durch hormonelle Präparate zur Konzeptionsverhütung gehemmt, so verhindern die in den hormonalen Kontrazeptiva selbst enthaltenen Estrogene das Auftreten von Estrogenmangelerschei ungen.

Im Falle klimakterischer Ausfallerscheinungen wird die Behandlung dieser Störungen üblicherweise durch die Anwendung natürlicher Estrogene angestrebt.

Diese können alleine in Intervallen gegeben werden oder zur Hemmung von Proliferationsvorgängen im Uterus in Kombination mit Gestagenen.

Estrogene haben ein breites Spektrum an sexualfunktionsspezifischen und metabolischen Funktionen.
Entsprechend breit ist das Spektrum unerwünschter Wirkungen, die mit der Anwendung von Estrogenen in verschiedenen Formen der Hormontherapie verbunden sein können. Der Krankheitswert entsprechender Störungen ist unterschiedlich. Nicht selten wird in der Anfangsphase einer Therapie das Auftreten von Übelkeit beobachtet, diese Beschwerden können im Verlauf der Behandlung verschwinden. Zu ernsteren Störungen können verschiedene Stoffwechselwirkungen führen. Es kommt unter Estrogenen zu Veränderungen von Faktoren, die die Blutgerinnung regulieren. Entsprechende Veränderungen können im Zusammenwirken mit verschiedenen anderen Faktoren (Beispiele: individuelle Disposition, Operationen, andere Traumen, Rauchen) thromboembolische Vorgänge begünstigen. Diese thrombischen Ereignisse können zu schweren Folgen wie Myokardinfarkt oder apoplektischer Insult führen.

Die angeführten Beispiele zeigen, daß es therapeutisch sinnvoll ist, Estrogenmangelzustände zu behandeln. Am überwiegen des Nutzens der Substititionstherapie besteht keinerlei Zweifel. Andererseits zeigen die bekannten Nebenwirkungen der hormonalen Kontrazeption, daß von therapeutisch zugeführten Estrogenen auch Gefahren ausgehen können.

Die Ursache aller beobachteten Nebenwirkungen ist in der Schwierigkeit zu sehen, bei den aus der Fachliteratur bekannten zuzuführenden Estrogenen eine über einen exakt definierten Wirkstoffspiegel letzlich definierte Wirkung im Organismus zu erzielen.

Nach Goldzieher et al. (American Journal of Ostetrics and Gynecology, Vol. 160/5, S. 1260-64, 1989), beispielsweise, traten nach der oralen Gabe einer definierten Dosis von Ethinylestradiol auch dann individuell sehr unterschiedliche Wirkstoffspiegel auf, wenn für die untersuchten Probandinnen die Bedingungen der Einnahme streng standardisiert waren.

Aus den festgestellten individuell variableren Blutkonzentrationen von Ethinylestradiol, die auch in anderen pharmakokinetischen Studien erhoben wurden, lassen sich Probleme ableiten, deren Reduktion Gegenstand der vorliegenden Erfindung ist.

Um einen bestimmten Blutspiegel von Ethinylestradiol zu erreichen, müßten Frauen mit unterschiedlichen Dosierungen dieses Estrogens behandelt werden. Dieses ist in der Praxis nicht möglich. Es ist die Annahme berechtigt, daß die in hormonalen Kontrazeptiva vorliegenden Dosierungen nur für einen bestimmten Anteil der Frauen, die diese Präparate anwenden, optimal sind. Für einen erheblichen Teil der Anwenderinnen dürfte die gegebene Dosis entweder zu hoch oder zu niedrig sein.

Die häufig beobachteten Durchbruch- und Zwischenblutungen unter Kontrazeptiva, sind ein Anhaltspunkt dafür, daß die betroffenen Frauen mit dem verwendeten Präparat eine zu niedrige Estrogendosis erhalten. Das Auftreten von Nebenwirkungen, die auf Estrogene zu beziehen sind, wie übelkeit, Brustspannen und die Retention von Flüssigkeit legen andererseits die Annahme nahe, daß in diesen Fällen die erreichten Estrogen-Konzentrationen oberhalb des optimalen Niveaus gelegen haben. Die geschilderte Verallgemeinerun9 erscheint zulässig, da Ethinylestradiol oder dessen Prodrug, das Mestranol, die einzigen Estrogene sind, die heute in kombinierten oralen Kontrazeptiva enthalten sind.

Für die in der klimakterischen Substitutionstherapie eingesetzten Substanzen besteht wegen ihrer insgesamt geringen und variablen oralen Bioverfügbarkeit ebenfalls die Schwierigkeit, die therapeutisch richtige Dosis exakt zu definieren.

Gyling et al. (J. Steroid. Biochem., 1988, 29(1), 1- 8) und Stoessel et al. (J. Steroid. Biochem., 1986, 25(5A), 677-682) beschreiben die Bindung an Rezeptoren bestimmter Estrogene und Antiestrogene.

14α, 15α-Methylenestradiol wird gleichfalls beschrieben.

Der Erfindung liegt die Aufgabe zugrunde, neue Wirkstoffe mit genau definierter estrogener Wirksamkeit zu finden.

Eine weitere Aufgabe der Erfindung ist es, Arzneimittel zur oralen Applikation der neuen Wirkstoffe zu finden.

Die Aufgabe wird erfindungsgemäß dadurch gelöst, daß 14α, 15α-Methylen-estradiol-Ester der allgemeinen Formel I 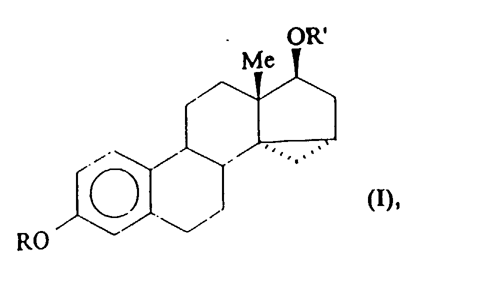 worin
- R: eine Gruppierung -COR¹,
wobei R¹
eine Alkyl-, Cycloalkyl-, Aryl- oder Dialkylamido-Gruppe darstellt,
sowie entweder
- R': ein Wasserstoffatom
oder
eine Gruppierung -COR¹,
wobei R¹
eine Cycloalkylgruppe darstellt,
bedeutet, geschaffen werden.

Die Herstellung der erfindungsgemäßen Ester erfolgt gemäß den Ansprüchen 2 bis 4.

Besondere Vorteile zeigen 14α, 15α-Methylen-estradiol-Ester als orale Arzneimittel in ihrer Verwendung als Kontrazeptiva und Antiklimakterika.

Beispielsweise können die erfindungsgemäßen Wirkstoffkombinationen vorzugsweise in Form von oral anwendbaren galenischen Zubereitungsformen appliziert werden. Als orale Zubereitungsformen kommen z.B. Tabletten, Dragees, Pillen oder Kapseln in Betracht. Die Zubereitungsformen werden in an sich üblicher Weise hergestellt, wie sie beispielsweise im "Remington's Pharmaceutical Sciences Handbook, Hack Pub. Co., N.Y., USA" beschrieben sind.

Die Vorteile der Erfindung ergeben sich im wesentlichen dadurch, daß 14α,15α-Methylen-estradiol-Ester gefunden wurden,
- die weniger Durchbruch- und Zwischenblutungen und weniger leichte und schwere Gesundheitsstörungen bewirken als die herkömmlichen Ethinylestradiol enthaltenden Kontrazeptiva, bedingt durch die Möglichkeit, die richtige Dosis exakt zu definieren,
- die durch die Möglichkeit der genauen Einstellung der Substitutionsdosis bei der klimakterischen Substitutionstherapie eine besser definierte Estrogenwirkung aufweisen, als das in der Substitutionstherapie verbreitete Estradiol,
- welche damit zu einer entscheidenden Verbesserung der Estrogentherapie beitragen
- und bei deren Anwendung ein größerer Kreis der Anwender innen, als bisher durch die unzureichende Möglichkeit des Einstellens eines bestimmten Blutspiegels, angesprochen werden kann.

Die vorteilhaften estrogen Wirkungen der erfindungsgemäßen Ester werden anhand folgender Verbindungen demonstriert:
17β-Hydroxy-14α,15α-methylen-estra-1,3,5(10)-trien-3-yl-benzoat (Code: J821)
17β-Hydroxy-14α,15α-methylen-estra-1,3,5(10)-trien-3-yl-cyclohexancarboxylat (Code: J883)
17β-Hydroxy-14α,15α-methylen-estra-1,3,5(10)-trien-3-yl-cyclopentancarboxylat (Code: J884)
17β-Hydroxy-14α,15α-methylen-estra-1,3,5(10)-trien-3-yl-pentanoat (Code: J 885)
14α,15α-Methylen-1,3,5(10)-trien-3,17β-diyl-dicyclo-hexancarboxylat (Code: J886)
17β-Hydroxy-14α,15α-methylen-estra-1,3,5(10)-trien-3-yl N,N-dimethylamidocarboxylat (Code: J893)

Als Vergleiche dienten Ethinylestradiol (EE) und 14α,15α-Methylen-estra-1,3,5(10)-trien-3,17β-diol (Code: J824) Die Ermittlung der Uterusfeuchtmassen nach einmaliger oraler Substanzapplikation sowie die Dosis-Wirkungsanalysen der Substanzen wurde zur Bemessung einer Estrogenwirkung herangezogen.

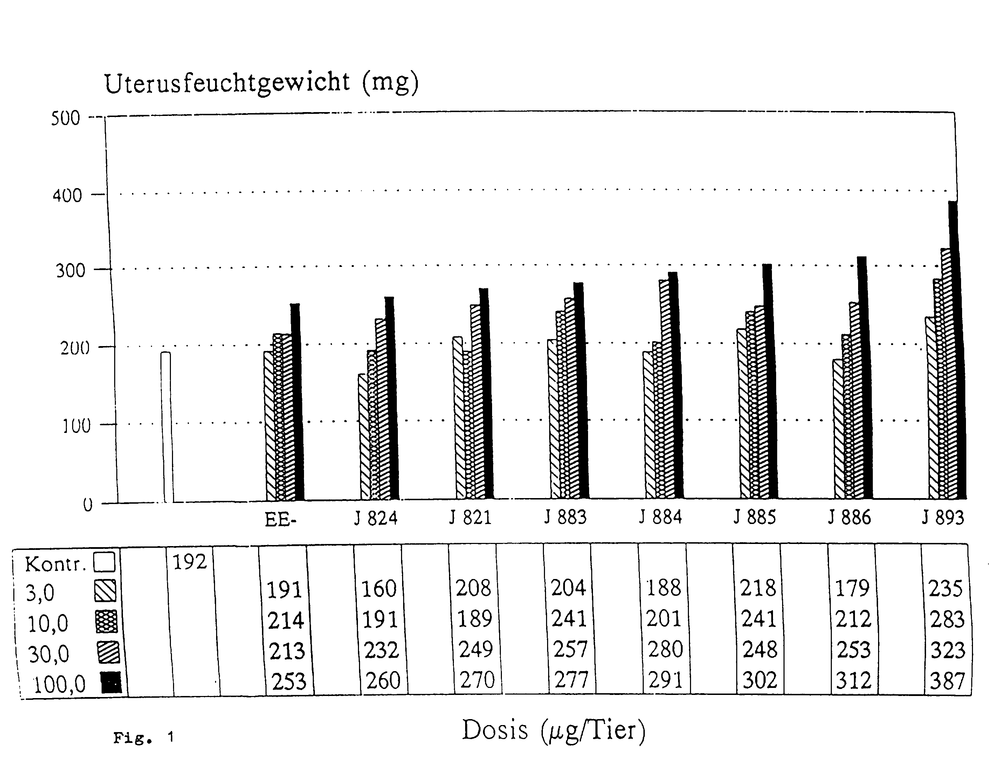

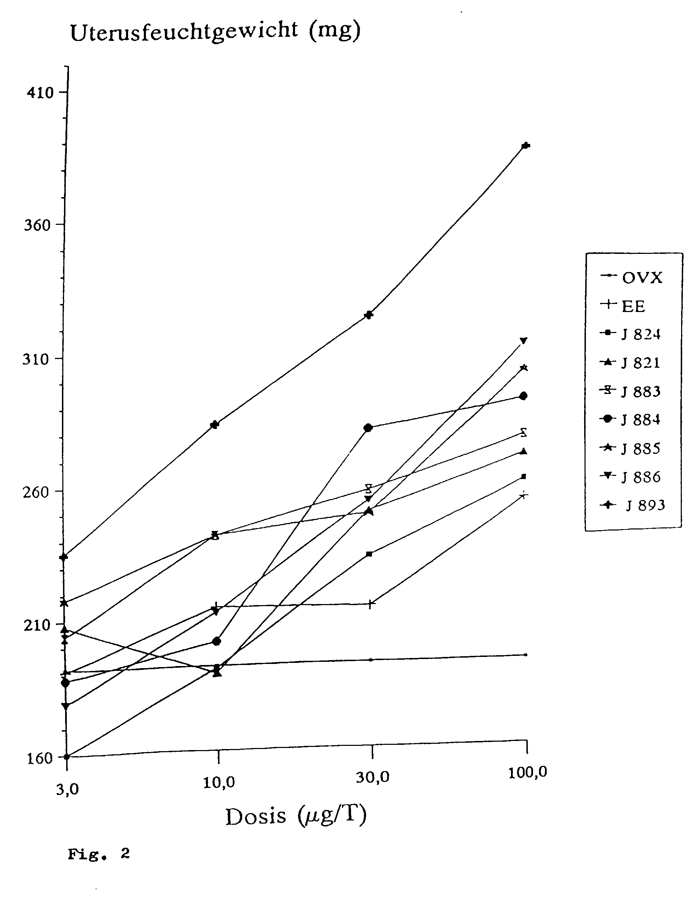

Abbildungen 1 und 2 zeigen, daß durch eine Veresterung der Grundstruktur J 824 die orale Estrogenwirkung verstärkt werden kann. Weiterhin ist die bessere estrogene Wirkung des J824 gegenüber EE ersichtlich.

Da die erfindungsgemäßen Verbindungen als Prodrugs von J 824 aufzufassen sind, aus denen J 824 als aktives Prinzip freigesetzt wird, ist die gefundene Überlegenheit im Vergleich zur Ausgangssubstanz nur über Änderungen pharmakokinetischer Einflußgrößen zu erklären. Es besteht eine statistisch gesicherte Überlegenheit der erfindungsgemäßen Verbindungen gegenüber der Ausgangssubstanz und gegenüber Ethinylestradiol. Besonders auffällig ist der steilere Verlauf der Dosis-Wirkungskurven der erfindungsgemäßen Verbindungen im Vergleich zu derjenigen von Ethinylestradiol (Abb. 2).

Der flachere Verlauf der Dosis-Wirkungskurve von Ethinylestradiol entspricht dem beim Menschen bekannten Phänomen individuell variierender pharmakokinetischer Parameter. Diese erklären die Existenz reagierender und nichtreagierender Individuen über einen breiten Dosisbereich.

Die beanspruchten Ester des 14α,15α-Methylenestradiols sind dem Ethinylestradiol und den natürlichen Estrogenen hinsichtlich oraler Wirksamkeit deutlich überlegene Substanzen.

Das beobachtete pharmakologische Verhalten zeigt, daß Ester des genannten Typs bzgl. Bioverfügbarkeit und estrogener Wirksamkeit besser definiert werden können als Ethinylestradiol bei oraler Anwendung. Entsprechend besitzen die genannten Ester Vorteile für die orale Estrogentherapie (Kontrazeption und klimakterische Substitutionstherapie).

Die Erfindung wird durch die nachfolgenden Ausführungsbeispiele näher erläutert.

### Beispiel 1

### 17β-Hydroxy-14α,15α-methylen-estra-1,3,5(10)-trien-3-yl Benzoat

a)Zu einer Suspension von 2g (7,03 mmol) 14α,15α-Methylenestra-1,3,5(10)-trien-3,17β-diol in 20 ml Chloroform werden unter Rühren nacheinander 2 ml Wasser, 1,5 ml (12,92mmol) Benzoylchlorid und 0,33 ml (2,38 mmol) Triethylamin zugefügt.
Nach Zugabe von 1 ml 90%iger Kalilauge wird für weitere 15 Minuten bei 20 bis 25°C gerührt und danach der Ansatz aufgearbeitet.
Hierzu wird die wässrige Phase von der organischen Phase abgetrennt und mit 5 ml Chloroform nachextrahiert. Die vereinigten organischen Phasen werden nacheinander mit verdünnter Salzsäure, Wasser, gesättigter wässriger Natriumhydrogencarbonatlösung und Wasser neutral gewaschen und danach im Vakuum zur Trockne eingeengt. Das erhaltene Rohprodukt wird an Kieselgel (Korngröße 0,063 bis 0,2 mm) chromatographiert. Nach Elution mit einem 1:1 Gemisch aus Toluen und Chloroform und anschließender Umkristallisation aus Aceton erhält man 17β-Hydroxy-14α,15α-methylen-estra-1,3,5(10)-trien-3-yl Benzoat vom Schmelzpunkt 180 bis 183 °C
[α]_{D}²⁰ :+97 ° (Chloroform ; c=1,0) ; UV λₘₐₓ nm (ε) : 231 (18765), Methanol.

b)Zu einer Lösung von 0,5 g (1,28 mmol) 14α,15α-Methylen-17-oxo-estra-1,3,5(10)-trien-3-yl Benzoat in 80 ml Methanol gibt man eine Lösung von 0,154 g (3,85 mmol) Natriumborhydrid in 1ml Wasser und rührt bei 20 bis 25°C insgesamt 5 Stunden. Danach verdünnt man den Reaktionsansatz mit 100 ml Wasser,säuert mit Essigsäure an und extrahiert mehrmals mit Chloroform. Die vereinigten organischen Phasen werden mit Wasser neutral gewaschen, über Natriumsulfat getrocknet und danach im Vakuum zur Trockne eingeengt.Das erhaltene Rohprodukt wird an Kieselgel (Korngröße 0,063 bis 0,2 mm) chromatographiert. Nach Elution mit Cyclohexan / Ethylacetat 7:3 und Umkristallisation aus Aceton erhält man 17β-Hydroxy-14α,15α-methylen-estra-1,3,5(10)-trien-3-yl Benzoat vom Schmelzpunkt: 179 bis 182 °C.

### Beispiel 2

### 17β-Hydroxy-14α,15α-methylen-estra-1,3,5(10)-trien-3-yl Cyclopentancarboxylat

Eine Suspension von 1 g (3,52 mmol) 14α,15α-Flethylen-estra-1,3,5(10)-trien-3,17β-diol in 10 ml Chloroform wird unter Rühren nacheinander mit 1 ml Wasser,1,6 ml (13,16 mmol) Cyclopentancarbonsäurechlorid, 0,17 ml (1,19 mmol) Triethylamin und 1,7 ml 90%iger Kalilauge versetzt. Man rührt den Ansatz weiter bei 20 bis 25 °C und kontrolliert den Reaktionsverlauf dünnschichtchromatographisch. Nach vollständigem Umsatz des Ausgangsmaterials wird wie unter den Bedingungen von Beispiel la aufgearbeitet. Nach Chromatographie des Rohproduktes an Kieselgel (Korngröße 0,063 bis 0,2 mm) und Elution mit Toluen/Ethylacetat/Chloroform 6:3:1 sowie nachfolgender Kristallisation aus Methanol erhält man 17β-Hydroxy-14α,15α-methylen-estra-1,3,5(10)-trien-3-yl Cyclopentancarboxylat vom Schmelzpunkt 130 bis 134°C; [α]_{D}²⁰:+108° (Chloroform; c=1,0); UV λₘₐₓ nm (ε) : 268 (799); 276 (780), Methanol.

### Beispiel 3

### 17β-Hydroxy-14α,15α-methylen-estra-1,3,5(10)-trien-3-yl Cyclohexancarboxylat

a)1,5g (5,27 mmol) 14α,15α-Methylen -estra-1,3,5(10)-trien-3,17β-diol, in 15 ml Chloroform suspendiert, werden unter Rühren nacheinander mit 1,5 ml Wasser, 1,32 ml (9,70 mmol) Cyclohexancarbonsäurechlorid, 0,25 ml (1,78 mmol) Triethylamin und 0,75 ml 90%iger Kalilauge versetzt. Man rührt den Ansatz weiter bei 20 bis 25 °C und kontrolliert den Reaktionsablauf dünnschichtchromatographisch. Nach vollständigem Umsatz wird wie unter den Bedingungen von Beispiel la aufgearbeitet. Nach Chromatographie des Rohproduktes an Kieselgel (Korngröße 0,063 bis 0,2 mm) und Elution mit Toluen/Ethylacetat/Chloroform 6:3:1 erhält man 17β-Hydroxy-14α,15α-methylen-estra-1,3,5(10)-trien-3-yl Cyclohexancarboxylat, das aus Methanol umkristallisiert wird.
Schmelzpunkt: 179 bis 181°C; [α]_{D}²⁵ : +98° (Chloroform; c=1,0);UV λₘₐₓ nm (ε) : 268 (789); 276 (789), Methanol.

b)Zu einer Lösung von 0,8 g (2,04 mmol) 14α,15α-Methylen 17- oxo-estra-1,3,5(10)-trien-3-yl Cyclohexancarboxylat in 100 ml Methanol gibt man 0,246 g (6,15 mmol)Natriumborhydrid in 1 ml Wasser und rührt bei 20 bis 25°C insgesamt 4 Stunden.

Danach verdünnt man den Reaktionsansatz mit Wasser, säuert mit Essigsäure an und extrahiert mit Chloroform. Nach weiterer Aufarbeitung und Chromatographie wie im Beispiel 1b beschrieben, erhält man nach Umkristallisation aus Methanol 17β-Hydroxy-14α,15α-methylen-estra-1,3,5(10)-trien-3-yl Cyclohexancarboxylat vom Schmelzpunkt 180 bis 182 °C.

### Beispiel 4

### 17β-Hydroxy-14α,15α-methylen-estra-1,3,5(10)-trien-3-yl Pentanoat

Eine Suspension von 1 g (3,52 mmol) 14α,15α-Flethylen-estra-1,3,5(10)-trien-3,17β-diol in 10 ml Dichlornethan wird unter Rühren nacheinander mit 2 ml Wasser, 0,084 g (0,368 mmol) Triethylbenzylammoniumchlorid (TEBAC) und 0,67 ml (5,75 mmol) Valeriansäurechlorid versetzt. Nach Zugabe von 1 ml 40%iger Natronlauge rührt man den Ansatz weiter bei 20 bis 25 °C und kontrolliert den Reaktionsverlauf dünnschichtchromatographisch. Nach vollständigem Umsatz des Ausgangsmaterials wird wie unter den Bedingungen von Beispiel la aufgearbeitet.
Nach Chromatographie des Rohproduktes an Kieselgel (Korngröße 0,063 bis 0,2 mm) und Elution mit Toluen/Ethylacetat/Chloroform 6:3:1 sowie nachfolgender Kristallisation aus Aceton/n-Hexan erhält man 17β-Hydroxy-14α,15α-methylen-estra-1,3,5(10)-trien-3-yl Pentanoat vom Schmelzpunkt 78 bis 82 °C.
[α]_{D}²⁵ :+104° (Chloroform; c=1,0);UV λₘₐₓ nm (ε) : 269 (811); 275 (811), Methanol.

### Beispiel 5

### 17β-Hydroxy-14α,15α-methylen-estra-1,3,5(10)-trien-3-yl N,N-Dimethylamidocarboxylat

1g (3,52 mmol) 14α,15α-Methylen-estra-1,3,5(10)-trien-3,17β-diol wird unter Rühren mit 50 ml Chloroform und 4,86 ml (35,2 mmol) Triethylamin versetzt. Zur erhaltenen klaren Lösung gibt man nacheinander 0,214 g (1,75 mmol) 4-( Dimethylamino)-pyridin und 3,22 ml (35,2 mmol) Dimethylcarbamoylchlorid zu und rührt anschließend drei Stunden bei 20 bis 25 °C. Nach Ablauf der Reaktionszeit verdünnt man den Reaktionsansatz mit 30 ml Wasser, trennt die organische Phase ab und wäscht diese nacheinander mit 5%iger Salzsäure, Wasser, gesättigter wässriger Natriumhydrogencarbonatlösung und Wasser neutral. Anschließend wird im Vakuum zur Trockne eingeengt. Das Rohprodukt wird aus Methanol umkristallisiert und man erhält
17β-Hydroxy-14α,15α-methylen-estra-1,3,5(10)-trien-3-yl N,N-Dimethylamidocarboxylat vom Schmelzpunkt 193 bis 196 °C; [α]_{D}²⁰ :+109° (Chloroform; c=1,0);
UV λₘₐₓ nm (ε) : 269 (818); 276 (782), Methanol.

### Beispiel 6

### 14α,15α-Methylen-estra-1,3,5(10)-trien-3,17β-diyl Dicyclohexancarboxylat

1g (3,52 mmol) 14α,15α-Methylen-estra-1,3,5(10)-trien-3,17β-diol in 10 ml Chloroform suspendiert, wird nacheinander unter Rühren mit 2 ml Wasser,1,76 ml (12,93 mmol) Cyclohexancarbonsäurechlorid,0,33 ml (2,35 mmol) Triethylamin und 1 ml 90%iger Kalilauge versetzt. Man rührt den Ansatz weitere 4 Stunden bei 20 bis 25 °C und kontrolliert den Reaktionsverlauf dünnschichtchromatographisch.
Nach vollständigem Umsatz wird wie unter den Bedingungen von Beispiel la aufgearbeitet.
Das erhaltene Rohprodukt wird in einem Methanol/Chloroform-Gemisch gelöst und bis zur beginnenden Kristallisation ein-geengt. Man erhält 14α,15α-Methylen-estra-1,3,5(10)-trien-3,17β-diyl Dicyclohexancarboxylat vom Schmelzpunkt 164 bis 168 °C; [α]_{D}²⁵ : + 60° (Chloroform; c=1,0);UV λₘₐₓ nm (ε) : 275 (808), Methanol.

### Untersuchungen zur biologischen Wirksamkeit von 14α,15α-Hethylen-estradiol-Estern

### Methodik

Sowohl Uterus als auch Vagina zeigen bei ovarektomierten Ratten eine von der Estrogendosis abhängige Gewichtszunahme. Diese ist auf eine Proliferation der Schleimhäute, erhöhte Proteinsynthese, aber auch auf eine Wassereinlagerung zurückzuführen.
Für die der Erfindung zu Grunde liegenden Untersuchungen wurden adulte ovarektomierte Ratten eingesetzt. In einer kontrollierten Studie wurden Ester des 14α,15α-Methylen-estradiols auf estrogene Wirkung bei oraler Applikation gegen Ethinylestradiol(EE) und 14α,15α-Methylen-estradiol (J 824) geprüft. Die angegebenen Dosierungen beziehen sich auf den Steroidkern der Moleküle. Die Testsubstanzen wurden einmalig oral appliziert. Als Parameter einer Estrogenwirkung wurden am 4. Tag des Versuches die Uterusgewichte ermittelt (Feuchtgewichte).

### Versuchstiere

Weibliche Ratten [Stamm: BOR:WISW (SPF/Cpb)], Körpermasse zu Versuchsbeginn 200 - 240 g wurden ovarektomiert und 10 Tage nach diesem Eingriff randomisiert den Versuchsgruppen zugeordnet. Dann erfolgte die einmalige orale Applikation (Tag 1) der Prüfsubstanzen.
Am 4. Tag nach der Applikation wurde die Sektion der Versuchstiere durchgeführt.
Tierzahl pro Gruppe: n = 7

### Tierhaltung

Die Tierhaltung erfolgte unter konventionellen Bedingungen (Raumtemperatur: 21 °C ± 2 °C; Lichtregime: 12 Stunden hell, 12 stunden dunkel). Die Tiere erhielten Haltungsdiät für Ratten und Mäuse von der Fa. Altromin sowie Trinkwasser ad libitum.

### Prüfsubstanzen

Ethinylestradiol (EE), J 824, J 821, J 883, J 884, J 885, J 886 und J 893 wurden in Myrj^{R} suspendiert.
Geprüft wurde in den Dosierungen 3 µg/T, 10µg/T, 30 µgtT und 100 µg/T. Die Dosisangabe bezieht sich auf den Steroidanteil der eingewogenen Substanzmenge. Die Kontrollgruppe erhielt nach Kastration eine einmalige Applikation des Substanzvehi kels Myrj^{R}.

### Bewertungsparameter

Die Ermittlung der Uterusfeuchtmassen nach einmaliger oraler Substanzapplikation wurden als Zeichen einer Estrogenwirkung gewertet.
- Ermittlung der Uterusgewichte und der daraus resultieren den relativen Estrogenität % zur Vehikelkontrolle
- Dosis-Wirkungsanalyse der Substanzen.

## Patentansprüche

1. 14α,15α-Methylen-estradiol-Ester
der allgemeinen Formel I 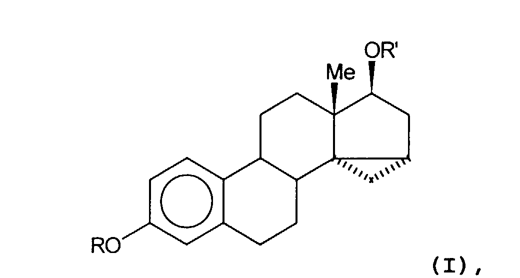 worin
R eine Gruppierung -COR¹,
wobei R¹
eine Alkyl-, Cycloalkyl-, Aryl- oder Dialkylamido-Gruppe darstellt,
sowie entweder
R' ein Wasserstoffatom
oder
eine Gruppierung -COR¹,
wobei R¹
eine Cycloalkylgruppe darstellt,
bedeutet.

2. Verfahren zur Herstellung von 14α,15α-Methylen-estradiol-Estern nach Anspruch 1, dadurch gekennzeichnet,
daß 14α,15α-Methylen-estradiol der Formel II 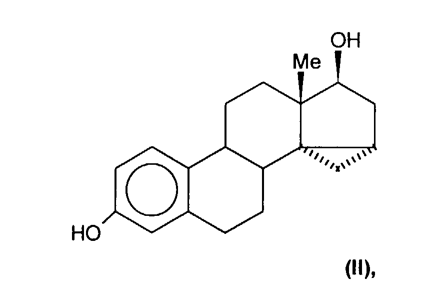 mit einem reaktiven Derivat einer Alkan-, Cycloalkan-, Aren- oder Dialkylamidocarbonsäure zur Reaktion gebracht wird.

3. Verfahren zur Herstellung von 14α,15α-Methylen-estradiol-Estern nach Anspruch 1, dadurch gekennzeichnet,
daß Ester des 14α,15α-Methylen-estrons der allgemeinen Formel III 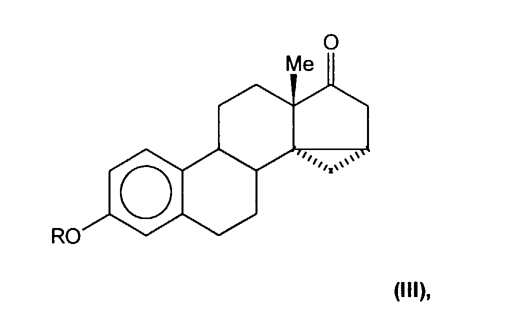
worin R eine Gruppierung -COR¹ ist, wobei R¹ die im Anspruch 1 angegebene Bedeutung hat, mit einem Reduktionsmittel in Ester des 14α,15α-Methylen-estradiols nach Anspruch 1,
worin für R eine Gruppierung -COR¹, wobei R¹ die im Anspruch 1 angegebene Bedeutung hat und für R' ein Wasserstoffatom stehen,
umgewandelt
und gegebenenfalls mit einem reaktiven Derivat einer Alkan-, Cycloalkan-, Aren- oder Dialkylamidocarbonsäure zur Reaktion gebracht werden.

4. Verfahren zur Herstellung von 14α,15α-Methylen-estradiol-Estern nach den Ansprüchen 2 bis 3,
dadurch gekennzeichnet,
daß die Veresterungen mit Hilfe der Phasentransfer-Katalyse vorgenommen werden.

5. Verwendung der 14α,15α-Methylen-estradiol-Ester nach Anspruch 1 zur Herstellung oraler Arzneimittel.

6. Verwendung der 14α,15α-Methylen-estradiol-Ester nach Anspruch 1 zur Herstellung von Kontrazeptiva und Antiklimakterika.

## Claims

1. 14α,15α-methylene estradiol ester of the general formula I 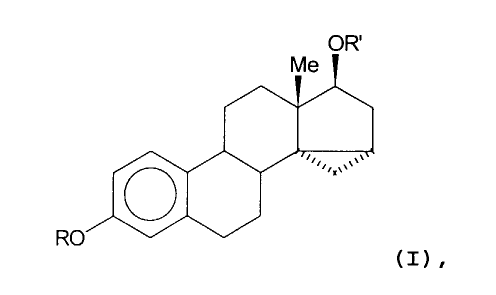 wherein
R is a -COR¹ grouping,
where R¹ represents an alkyl, cycloalkyl, aryl or dialkylamido group,
and
R' represents
either a hydrogen atom
or a -COR¹ grouping,
wherein R¹ represents a cycloalkyl group.

2. A method for the production of 14α,15α-methylene estradiol ester according to claim 1, characterized in that 14α,15α-methylene estradiol of the formula II 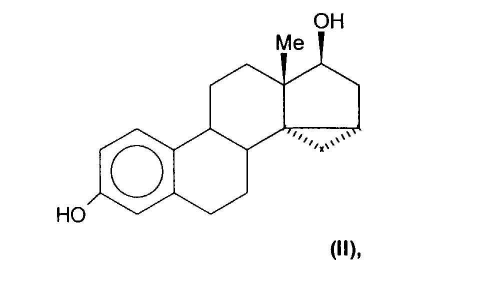 is reacted with a reactive derivative of an alkane carboxylic acid, cycloalkane carboxylic acid, aromatic carboxylic acid or dialkylamido carboxylic acid.

3. A method for the production of 14α,15α-methylene estradiol ester according to claim 1, characterized in that an ester of a 14α,15α-methylene estrone of the general formula III 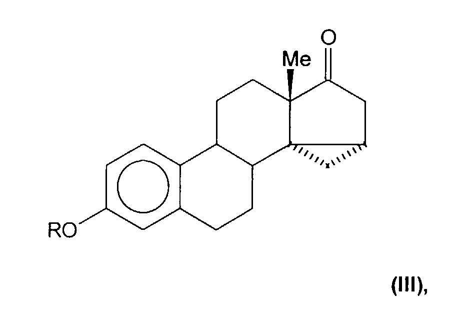 wherein
R is a -COR¹ grouping, wherein R¹ has the meaning as given in claim 1
is converted by a reductant into an ester of 14α,15α-methylene estradiol according to claim 1, wherein R is a -COR¹ grouping, wherein R¹ has the meaning as given in claim 1 and R' represents a hydrogen atom,
and may optionally be reacted with a reactive derivate of an alkane carboxylic acid, cycloalkane carboxylic acid, aromatic carboxylic acid or dialkylamido carboxylic acid.

4. A method for the production of 14α,15α-methylene estradiol ester according to claims 2 to 3,
characterized in that the esterification is carried out using phase transfer catalysis.

5. Use of 14α,15α-methylene estradiol ester according to claim 1 for the production of oral pharmaceuticals.

6. Use of 14α,15α-methylene estradiol ester according to claim 1 for the production of contraceptives and for pharmaceuticals for treating the symptoms of menopause.

## Revendications

1. Ester de 14α, 15α-méthylène-oestradiol de formule générale I 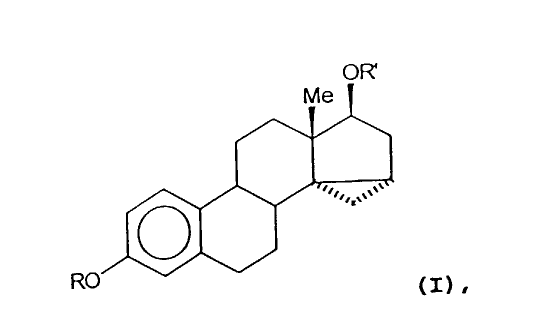 dans laquelle
R représente un groupe -COR¹,
dans lequel R¹ représente un groupe alkyle, cycloalkyle, aryle ou dialkylamido,
et R' représente un atome d'hydrogène
ou
un groupe -COR¹,
R¹ représentant un groupe cycloalkyle.

2. Procédé de fabrication d'esters de 14α, 15α-méthylène-oestradiol selon la revendication 1, caractérisé en que le 14α, 15α-méthylène-oestradiol de formule II 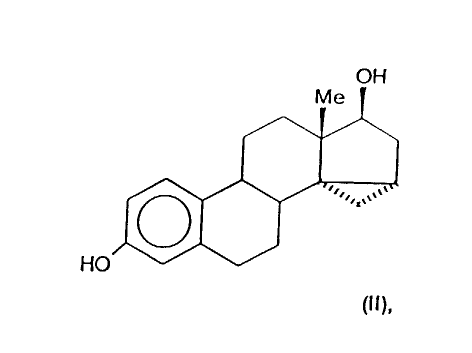 est mis à réagir avec le dérivé réactif d'un acide alkyl-, cycloalkyl-, aryl- ou dialkylamidocarboxylique.

3. Procédé de fabrication d'esters de 14α, 15α-méthylène-oestradiol selon la revendication 1, caractérisé en que l'ester de la 14α, 15α-méthylène-oestrone de formule générale III 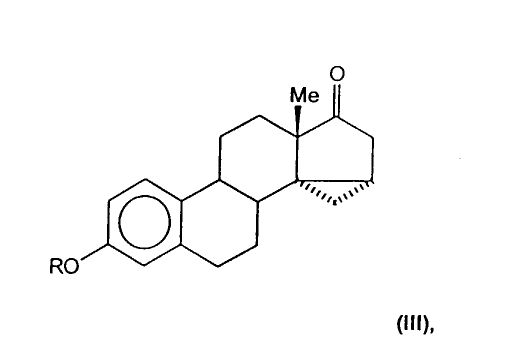
dans laquelle R est un groupe -COR¹, dans lequel R¹ a la signification donnée dans la revendication 1,
est transformé à l'aide d'un moyen de réduction en ester de 14α, 15α-méthylène-oestradiol selon la revendication 1, R représentant un groupe -COR¹ dans lequel R¹ a la signification donnée dans la revendication 1 et R' étant un atome d'hydrogène
et est, le cas échéant, mis à réagir avec un dérivé réactif d'un acide alkyl-, cycloalkyl-, aryl- ou dialkylamidocarboxylique.

4. Procédé de fabrication d'esters de 14α, 15α-méthylène-oestradiol selon les revendications 2 à 3, caractérisé en que l'estérification est menée à l'aide d'une catalyse de transfert de phases.

5. Utilisation de l'ester de 14α, 15α-méthylène-oestradiol selon la revendication 1 pour la fabrication de médicament à administration orale.

6. Utilisation de l'ester de 14a, 15α-méthylène-oestradiol selon la revendication 1 pour la fabrication d'un contraceptif et d'un médicament destiné à lutter contre les effets de la ménopause.
